# EUROPEAN PATENT APPLICATION

(11) **EP 4 205 558 A1**
(43) Date of publication of application: **05.07.2023**
(21) Application number: 20951650.9
(22) Date of filing: 31.08.2020
(51) Int. Cl.: A23L 33/105, A61K 36/287, A61P 29/00, A61P 25/28, A61P 39/06

(54) **HEALTH FUNCTIONAL FOOD, COMPRISING SIBERIAN CHRYSANTHEMUM EXTRACT, FOR PAIN RELIEF OR ANTIOXIDATION**

(71) Applicant: Green Cross WellBeing Corporation, Yeongdeungpo-gu, Seoul 07335 (KR)
(72) Inventor: HAN, Hae Jung, Seongnam-si Gyeonggi-do 13595 (KR); YOO, Young Hyo, Seongnam-si Gyeonggi-do 13595 (KR); YUN, Jong Bok, Seongnam-si Gyeonggi-do 13595 (KR); KIM, Joo Young, Seongnam-si Gyeonggi-do 13595 (KR); PARK, Sun Kyu, Seongnam-si Gyeonggi-do 13595 (KR)
(74) Representative: dompatent von Kreisler Selting Werner - Partnerschaft von Patent- und Rechtsanwälten mbB
(86) International application number: PCT/KR2020/011644
(87) International publication number: WO 2022/045418

(57) **Abstract**

The present invention relates to a health functional food for pain relief or antioxidation, including Chrysanthemum Sibiricum extract, and a pharmaceutical composition for treatment of relates diseases, with advantages in that reactive oxygen species-induced oxidative damage is improved thus to prevent and enhance related diseases, effects of relieving pains induced by different causes are proved, thereby being useable as an analgesic.

## Description

### [Technical Field]

The present invention relates to a health functional food for pain relief or antioxidation, including Chrysanthemum Sibiricum extract.

### [Background Art]

Synovial cells are cells of the synovial membrane that cover an inner surface of the joint cavity, and are not epithelial but connective tissue cells which are divided into two types, A and B. Type A cells are in the form of macrophages, have phagocytosis, and form many Filopodia on the surface thereof, whereas type B cells are fibroblasts with a relatively smooth cell surface and contain a lot of rough endoplasmic reticulum in the cytoplasm.

Reactive oxygen species (ROS) including peroxide anions, hydrogen peroxide and hydroxyl radicals, etc. derived from oxygen, are required for general functions involved in regulating intracellular signals and redox reactions, but excessive ROSs are known to induce oxidative stress, thereby accelerating tumor development and aging.

Chrysanthemum Sibiricum is a perennial herb in the Asteraceae family that grows wild at the foot of mountains, riversides, and fields across the country, and is distributed in Korea, northern China, Manchuria, and Siberia. It is known to have hot properties and strong anti-inflammatory and analgesic effects. Therefore, according to herbal remedy and home remedy, the entire Chrysanthemum herb with flowers thereof gathered and dried in the shade has been used as a home medicine for women who have cold hands and feet or a cold body after childbirth, and is also known to be effective in treating hypertension and gastrointestinal diseases.

### [Summary of Invention]

### [Problems to be Solved by Invention]

It is an object of the present invention to provide a health functional food having pain relief or antioxidant effects, including Chrysanthemum Sibiricum extract ("Chrysanthemum extract").

Another object of the present invention is to provide a pharmaceutical composition having pain relief or antioxidant effects, including Chrysanthemum extract.

### [Means for Solving Problems]

1. A health functional food for pain relief or antioxidation, including Chrysanthemum Sibiricum extract ("Chrysanthemum extract").
2. The health functional food according to the above 1, wherein the antioxidation is to improve oxidative damage of synovial cells.
3. The health functional food according to the above 2, wherein the oxidative damage is damage in a patient with degenerative arthritis.
4. The health functional food according to the above 1, wherein the food is used to prevent or improve a disease selected from the group consisting of: Alzheimer's disease, Parkinson's disease, Huntington's disease, amyotrophic lateral sclerosis, multiple sclerosis, obesity, hyperglycemia, dyslipidemia, hypertension, diabetes mellitus, heart failure, cardiovascular ischemia, cerebral ischemic injury, stroke, myocardial infarction, systemic lupus erythematosus, COPD, asthma, acute kidney injury, chronic kidney disease, diabetic nephropathy, end-stage renal disease, viral hepatitis, liver cirrhosis, inflammatory bowel disease, viral infectious disease, solid cancer, hematologic cancer, sepsis, recurrent polychondritis, Kienbock's disease in adults, osteochondrosis of the carpal meniscus, complex regional pain syndrome, Paget's disease, progressive ossified fibrodysplasia, relapsing steatosis, multifocal fibrosclerosis, diffuse Fasciitis, polymyalgia rheumatica, Behcet's disease, mixed connective tissue disease, Sjegren's syndrome, dry syndrome, systemic sclerosis, Crest's syndrome, polymyositis, dermatomyositis, systemic lupus erythematosus, aortic arch syndrome, Wegener's granulomatosis, lethal median granuloma, thrombotic thrombocytopenic purpura, thrombotic microangiopathy, Goodpasture's syndrome, polyarteritis, polyarthritis, juvenile arthritis, juvenile rheumatoid spondylitis, rheumatoid arthritis and adult-onset Still's disease.
5. The health functional food according to the above 1, wherein the Chrysanthemum extract is a hot water extract or an organic solvent extract.
6. The health functional food according to the above 1, wherein the pain is selected from the group consisting of: somatic pain, visceral pain, inflammatory pain, dysfunctional pain, idiopathic pain, neuropathic pain, superficial pain, deep pain, itching, migraine and cancer pain.
7. A pharmaceutical composition for treatment or prevention of a disease, including Chrysanthemum Sibiricum extract ("Chrysanthemum extract"), wherein the disease is selected from the group consisting of: Alzheimer's disease, Parkinson's disease, Huntington's disease, amyotrophic lateral sclerosis, multiple sclerosis, obesity, hyperglycemia, dyslipidemia, hypertension, diabetes mellitus, heart failure, cardiovascular ischemia, cerebral ischemic injury, stroke, myocardial infarction, systemic lupus erythematosus, COPD, asthma, acute kidney injury, chronic kidney disease, diabetic nephropathy, end-stage renal disease, viral hepatitis, liver cirrhosis, inflammatory bowel disease, viral infectious disease, solid cancer, hematologic cancer, sepsis, recurrent polychondritis, Kienbock's disease in adults, osteochondrosis of the carpal meniscus, complex regional pain syndrome, Paget's disease, progressive ossified fibrodysplasia, relapsing steatosis, multifocal fibrosclerosis, diffuse Fasciitis, polymyalgia rheumatica, Behcet's disease, mixed connective tissue disease, Sjegren's syndrome, dry syndrome, systemic sclerosis, Crest's syndrome, polymyositis, dermatomyositis, systemic lupus erythematosus, aortic arch syndrome, Wegener's granulomatosis, lethal median granuloma, thrombotic thrombocytopenic purpura, thrombotic microangiopathy, Goodpasture's syndrome, polyarteritis, polyarthritis, juvenile arthritis, juvenile rheumatoid spondylitis, rheumatoid arthritis and adult-onset Still's disease.
8. The pharmaceutical composition according to the above 7, wherein the pain is selected from the group consisting of: somatic pain, visceral pain, inflammatory pain, dysfunctional pain, idiopathic pain, neuropathic pain, superficial pain, deep pain, itching, migraine and cancer pain.
9. The pharmaceutical composition according to the above 7, wherein the prevention or treatment is based on antioxidation by reduction of Thiobarbituric acid reactive substance (TBARS).
10. The pharmaceutical composition according to the above 9, wherein the antioxidation is to improve oxidative damage of synovial cells.
11. The pharmaceutical composition according to the above 7, wherein the Chrysanthemum extract is a hot water extract or an organic solvent extract.

### [Advantageous effects]

The health functional food of the present invention includes Chrysanthemum extract to exhibit pain relief or antioxidant effects.

Further, the pharmaceutical composition of the present invention includes Chrysanthemum extract to exhibit therapeutic effects on diseases caused by pain and oxidative damage.

### [Brief Description of Drawings]

FIG. 1 is a schematic diagram illustrating a timeline of human body application test.
FIG. 2 illustrates identification of analgesic efficacy by comparison of the response time after administration according to the dose of Chrysanthemum extract in a hot plate mouse analgesic model.
FIG. 3 is a schematic diagram illustrating the timeline of acetic acid-induced analgesic effect experiments.
FIG. 4 illustrates a graph counting the number of occurrences of writhing syndrome after administration according to the dose of Chrysanthemum extract.

### [Mode for Carrying out Invention]

Hereinafter, the present invention will be described in detail.

The present invention relates to a health functional food for pain relief or antioxidation, which includes Chrysanthemum Sibiricum extract.

"Gujeolcho" is a resource plant native to Korea and refers to Chrysanthemum Sibiricum in Chrysanthemum family, and specifically, may include Chrysanthemum Sibiricum ("Chrysanthemum") or similar plants such as Pocheon dendranthema, Halla dendranthema, Nakdong dendranthema, Rocky dendranthema, etc., but it is not limited thereto. It doesn't matter that any part of Chrysanthemum such as flowers, leaves, stems and roots is used.

In the present invention, the extract means an active ingredient separated from a natural product, is prepared by an extraction process using water, an organic solvent or a mixed solvent thereof, and may include an extract obtained using water, an organic solvent or a mixed solvent thereof, dried powder thereof, or all formulated forms prepared using the same. Further, the extract may also include fractions of the extract that has undergone the extraction process.

The Chrysanthemum extract may be extracted by any method known in the art, for example, may be a hot water extract or an ultrasonic extract.

The hot water extract may be extracted, for example, with hot water of 40 to 120°C, but it is not limited thereto.

During hot water extraction, water may be added, for example, in an amount of 2 to 20 times a weight of Chrysanthemum, but it is not limited thereto.

The heating time at the hot water extraction may be, for example, 1 to 10 hours, specifically 2 to 5 hours, more specifically 2 to 4 hours, and most specifically 2 to 3 hours, but it is not limited thereto.

The hot water extract may be obtained by filtration to produce a supernatant, followed by further performing steps such as concentration and lyophilization.

The ultrasonic extract may be obtained by immersing the Chrysanthemum in ethanol at a concentration of 10 to 95% and then extraction using an ultrasonic extractor, wherein the ultrasonic wave may, for example, range from 10 to 100 kHz, specifically 20 to 80 kHz, and more specifically 20 to 70 kHz, but it is not limited thereto.

The solvent used for the preparation of the extract may be water, an organic solvent, or a mixture thereof, specifically water, lower alcohol having 1 to 4 carbon atoms, acetone, petroleum ether, ethyl acetate, butyl acetate, trichloromethane, dichloromethane, chloroform, hexane or 1,3-butylene glycol, and specifically may be ethanol, but it is not limited thereto.

In the present invention, antioxidation may be to improve oxidative damage of synovial cells.

Synovial cells refer to cells of the synovial membrane that covers an inner surface of the joint cavity, and the health functional food of the present invention may improve oxidative damage to the synovial cells that may occur due to oxidative stress of reactive oxygen species (ROS) .

The oxidative damage to the synovial cells may be, for example, oxidative damage to synovial cells in a patient with degenerative arthritis, but it is not limited thereto.

Further, the health functional food of the present invention may have excellent antioxidant effects, thus to exhibit performance of preventing or improving various diseases related to oxidation. For example, the health functional food of the present invention may exhibit prevention or improvement performance to the disease selected from the group consisting of: Alzheimer's disease, Parkinson's disease, Huntington's disease, amyotrophic lateral sclerosis, multiple sclerosis, obesity, hyperglycemia, dyslipidemia, hypertension, diabetes mellitus, heart failure, cardiovascular ischemia, cerebral ischemic injury, stroke, myocardial infarction, systemic lupus erythematosus, COPD, asthma, acute kidney injury, chronic kidney disease, diabetic nephropathy, end-stage renal disease, viral hepatitis, liver cirrhosis, inflammatory bowel disease, viral infectious disease, solid cancer, hematologic cancer, sepsis, recurrent polychondritis, Kienbock's disease in adults, osteochondrosis of the carpal meniscus, complex regional pain syndrome, Paget's disease of unspecified bone, Paget's disease of other bones, Paget's disease of the skull, progressive ossified fibrodysplasia, relapsing steatosis, multifocal fibrosclerosis, diffuse Fasciitis, polymyalgia rheumatica, Behcet's disease, mixed connective tissue disease, Sjegren's syndrome with myopathy, dry syndrome, Sjegren's syndrome with keratoconjunctivitis, Sjegren's syndrome with lung involvement, Sjegren's syndrome with renal tubule-interstitial disorder, systemic sclerosis with pulmonary involvement, systemic sclerosis with myopathies, Crest's syndrome, combination of calcification-Raynaud's phenomenon-esophageal dysfunction-sclerosis dactylitis-capillary dilatation, progressive systemic sclerosis, polymyositis, other dermatomyositis, juvenile dermatomyositis, systemic lupus erythematosus with organ or systemic involvement, microscopic polyarteritis, aortic arch syndrome, Wegener's granulomatosis, lethal median granuloma, thrombotic thrombocytopenic purpura, thrombotic microangiopathy, Goodpasture's syndrome, juvenile polyarteritis, polyarteritis with pulmonary involvement, polyarteritis nodosa, juvenile polyarthritis, chronic juvenile polyarthritis, systemically onset juvenile arthritis, juvenile rheumatoid spondylitis, juvenile rheumatoid arthritis with or without a rheumatoid factor, juvenile rheumatoid arthritis and adult-onset Still's disease.

Paget's disease may include, for example, Paget's disease of unspecified bones, Paget's disease of other bones, or Paget's disease of the skull, but it is not limited thereto.

Sjegren's syndrome may include, for example, Sjegren's syndrome with myopathy, Sjegren's syndrome with keratoconjunctivitis, Sjegren's syndrome with pulmonary involvement, or Sjegren's syndrome with renal tubule-interstitial disorder, but it is not limited thereto.

The systemic sclerosis may include, for example, systemic sclerosis with pulmonary involvement, systemic sclerosis with myopathy, or progressive systemic sclerosis, but it is not limited thereto.

Crest syndrome may include, for example, a combination of calcification, Raynaud's phenomenon, esophageal dysfunction, sclerosis dactylitis and capillary dilatation, but it is not limited thereto.

The dermatomyositis may include, for example, other dermatomyositis or juvenile dermatomyositis, but it is not limited thereto.

The systemic lupus erythematosus may include, for example, systemic lupus erythematosus with organ or systemic involvement, but it is not limited thereto.

The polyarteritis may include, for example, microscopic polyarteritis, juvenile polyarteritis, polyarteritis with pulmonary involvement, or polyarteritis nodosa, but it is not limited thereto.

The polyarthritis may include, for example, juvenile polyarthritis or chronic juvenile polyarthritis, but it is not limited thereto.

The juvenile arthritis may include, for example, systemically onset juvenile arthritis, but it is not limited thereto.

The rheumatoid arthritis may include, for example, juvenile rheumatoid arthritis with or without a rheumatoid factor, or juvenile rheumatoid arthritis, but it is not limited thereto.

Among the diseases listed above, recurrent polychondritis, Kienbock's disease in adults, osteochondrosis of the carpal meniscus, complex regional pain syndrome, Paget's disease of unspecified bone, Paget's disease of other bones, Paget's disease of the skull, progressive ossified fibrodysplasia, relapsing steatosis, multifocal fibrosclerosis, diffuse Fasciitis, polymyalgia rheumatica, Behcet's disease, mixed connective tissue disease, Sjegren's syndrome with myopathy, dry syndrome, Sjegren's syndrome with keratoconjunctivitis, Sjegren's syndrome with lung involvement, Sjegren's syndrome withrenal tubule-interstitial disorder, systemic sclerosis with pulmonary involvement, systemic sclerosis with myopathies, Crest's syndrome, combination of calcification-Raynaud's phenomenon-esophageal dysfunction-sclerosis dactylitis-capillary dilatation, progressive systemic sclerosis, polymyositis, other dermatomyositis, juvenile dermatomyositis, systemic lupus erythematosus with organ or systemic involvement, microscopic polyarteritis, aortic arch syndrome, Wegener's granulomatosis, lethal median granuloma, thrombotic thrombocytopenic purpura, thrombotic microangiopathy, Goodpasture's syndrome, juvenile polyarteritis, polyarteritis with pulmonary involvement, polyarteritis nodosa, juvenile polyarthritis, chronic juvenile polyarthritis, systemically onset juvenile arthritis, juvenile rheumatoid spondylitis, juvenile rheumatoid arthritis with or without a rheumatoid factor, juvenile rheumatoid arthritis and adult-onset Still's disease may be rare diseases designated by the Korea Centers for Disease Control and Prevention ("KCDC"), and details of such rare diseases are described in rare disease helpline of KCDC, but they are not limited thereto.

The diseases listed above refer to diseases caused by oxidative damage, and in addition to the above listed diseases, diseases that are generally known to be caused by oxidative damage, for example, neurodegenerative diseases, metabolic syndrome, cardiovascular disorders, autoimmune diseases, inflammatory lung disease, kidney disease, liver disease, digestive disease, viral infectious disease, cancer, other inflammatory diseases, or the like may also be included, but they are not limited thereto.

Antioxidation may be expressed, for example, by Thiobarbituric acid reactive substance (TBARS) or by reduction of Matrix Metalloproteinase (MMP).

A level of Thiobarbituric acid reactive substance (TBARS) is used to determine the improvement of oxidative damage, and TBARS is a substance produced by reacting Malondialdehyde (MDA) generated by peroxidation of various biological membranes with 2-thiobarbituric acid (TBA). As an indicator related to cell damage caused by free radicals, it can be determined that the higher the level of TBARS, the greater the oxidative damage.

Further, in order to determine the improvement of the oxidative damage, a level of matrix metalloproteinase (MMP) may be checked. Since MMP expression is activated when active oxygen is increased due to oxidative damage, it can be determined that oxidative damage is improved when the level of MMP is decreased, specifically MMP-3.

In the present invention, "pain" may include somatic pain, visceral pain, inflammatory pain, dysfunctional pain, idiopathic pain, neuropathic pain, superficial pain, deep pain, itching, migraine and cancer pain, but it is not limited thereto.

Further, the present invention relates to a pharmaceutical composition including Chrysanthemum Sibiricum extract in order to treat or prevent a disease selected from the group consisting of: pain, Alzheimer's disease, Parkinson's disease, Huntington's disease, amyotrophic lateral sclerosis, multiple sclerosis, obesity, hyperglycemia, dyslipidemia, hypertension, diabetes mellitus, heart failure, cardiovascular ischemia, cerebral ischemic injury, stroke, myocardial infarction, systemic lupus erythematosus, COPD, asthma, acute kidney injury, chronic kidney disease, diabetic nephropathy, end-stage renal disease, viral hepatitis, liver cirrhosis, inflammatory bowel disease, viral infectious disease, solid cancer, hematologic cancer and sepsis.

The pain may be a pain within the range exemplified above, but it is not limited thereto.

Antioxidation may be expressed, for example, by Thiobarbituric acid reactive substance (TBARS) or by reduction of Matrix Metalloproteinase (MMP).

The level of Thiobarbituric acid reactive substance (TBARS) is used to determine the improvement of oxidative damage, and TBARS is a substance produced by reacting Malondialdehyde (MDA) generated by peroxidation of various biological membranes with 2-thiobarbituric acid (TBA). As an indicator related to cell damage caused by free radicals, it can be determined that the higher the level of TBARS, the greater the oxidative damage.

Further, in order to determine the improvement of the oxidative damage, the level of matrix metalloproteinase (MMP) may be checked. Since MMP expression is activated when active oxygen is increased due to oxidative damage, it can be determined that oxidative damage is improved when the level of MMP is decreased, specifically MMP-3.

The pharmaceutical composition of the present invention may be for improving oxidative damage of synovial cells, and the improvement of oxidative damage of synovial cells may be within the above-described exemplary range, but it is not limited thereto.

The Chrysanthemum extract included in the pharmaceutical composition of the present invention may be a hot water extract, an organic solvent extract or an ultrasonic extract, but it is not limited thereto.

The health functional food or pharmaceutical composition of the present invention may include Chrysanthemum extract granules prepared by mixing the Chrysanthemum extract with a dextrin-based compound.

The dextrin-based compound serves to change properties of the Chrysanthemum extract basically having a high viscosity (e.g., starch syrup-like form) into granular properties, and may be used in an amount of 50 to 70% by weight ("wt. %"), preferably 55 to 65 wt.%, and more preferably about 60 wt.% based on a total weight of the Chrysanthemum extract granules. Meanwhile, as used herein, the term "granules" may include fine powder, specifically, a mixture of granular particles and fine powder particles, or may be formed by agglomerating fine powder particles. In this case, a size of the granular particles is not particularly limited as long as it can achieve the purpose of the present invention to improve stability such as a particle size of pharmaceuticals generally manufactured in the art.

If a content of the dextrin-based compound is less than 50 wt.% based on the total weight of the Chrysanthemum extract granules, a raw material may be prepared in the form of wet (containing large moisture) granules or powder compared to the existing ones. Therefore, when a finished medicine product is manufactured using the raw material, a binding property of the raw material may be increased to reduce anti-deliquescent effects of a natural extract, thereby delaying a disintegration time. Further, if the dextrin-based compound exceeds 70 wt.% based on the total weight of the Chrysanthemum extract granules, a ratio of the Chrysanthemum extract is relatively decreased, such that a large amount of mixture (Chrysanthemum extract + dextrin-based compound) during a formulation test, and as a result, there are problems that tablet formation is reduced and the disintegration time, etc. is altered during production of the finished medicine.

Such a dextrin-based compound may include, for example, maltodextrin, cyclodextrin, other dextrin-based compounds having properties and features similar or identical to the above materials, and a mixture thereof, and it may be preferable to use maltodextrin in consideration of economical aspect.

The health functional food of the present invention may include general food additives in addition to the above-described Chrysanthemum extract or its granules, and whether or not it is suitable as a food additive may be determined by standards and criteria for corresponding items according to general rules and general test methods in the Food Additives Code approved by the Ministry of Food and Drug Safety, or the like, unless otherwise specified.

Examples of the items listed in the Food Additives Code may include chemical compounds such as ketones, glycine, calcium citrate, nicotinic acid, and cinnamic acid; natural additives such as persimmon pigment, licorice extract, crystalline cellulose, millet pigment, and guar gum; mixed preparations such as sodium L-glutamate preparation, alkali agent added to noodles, preservative agent, tar color agent, etc., but they are not limited thereto.

For example, a health functional food in a tablet form may be formed by granulating a mixture of the extract and an excipient, a binder, a disintegrant and other additives in a conventional manner, followed by compression-molding along with addition of a lubricant, or may be formed by directly compression-molding the mixture. Further, the health functional food in the tablet form may contain a flavoring agent, if necessary.

Among health functional foods in the form of capsules, a hard capsule agent may be prepared by filling a mixture of the extract and additives such as excipients in a conventional hard capsule, while a soft capsule agent may be prepared by filling a mixture of the extract and additives such as excipients in a capsule base such as gelatin. The soft capsule agent may further contain a plasticizer such as glycerin or sorbitol, a colorant, a preservative, and the like, if necessary.

Further, a health functional food in the form of pills may be prepared by molding a mixture of the extract and an excipient, a binder, a disintegrant, etc. according to any existing method known in the art. Further, if necessary, it may be coated with sucrose or other coating agents, or may be surface-coated with a material such as starch, talc, etc.

Further, a health functional food in the form of granules may be prepared by processing a mixture of the extract and an excipient, a binder, a disintegrant, etc. into granules according to any existing method known in the art, and may contain a fragrance agent or a flavoring agent, etc. if necessary.

The health functional food may include, for example, beverages, meat, chocolate, foods, confectionery, pizza, ramen, other noodles, gums, candies, ice cream, alcoholic beverages, vitamin complexes, health supplements and the like.

The health functional food may be orally applied for the purpose of nutritional supplements, and the application form is not particularly limited. For example, in the case of oral administration, the daily intake is preferably 5000 mg or less, more preferably 2000 mg or less, and most preferably 1000 mg or less. When formulated as a capsule or tablet, one (1) tablet may be administered with water once a day.

The pharmaceutical composition of the present invention may further include crystalline cellulose in addition to the above-described Chrysanthemum extract granules (formed of Chrysanthemum extract and dextrin-based compound). The crystalline cellulose is a component that can be used together with a lubricant to be described below to ensure stability against moisture. Therefore, when medicating the Chrysanthemum extract according to the present invention, the tablet coating corresponds to a general coating rather than a moisture-proof coating. The crystalline cellulose may be used in an amount of 50 to 70 wt.%, preferably 50 to 65 wt.%, and more preferably 50 wt.% or more and less than 60 wt.% based on the total weight of the pharmaceutical composition of the present invention. If a total weight of an initial finished composition is rather high when considering a size of tablets, a content or weight of the crystalline cellulose and a lubricant described below may be reduced. That is, a ratio of additives may be adjusted in consideration of the tablet size.

If the content of the crystalline cellulose is less than 50 wt.% based on the total weight of the pharmaceutical composition of the present invention, the disintegration time may be delayed compared to the existing composition. Further, anti-deliquescent effects may also be decreased, such that a change in stability due to the increased water content may occur. Further, when the content of the crystalline cellulose exceeds 70 wt.% based on the total weight of the pharmaceutical composition of the present invention, a ratio of the active ingredient in the mixture of the same weight is relatively reduced due to an increase in the content of the crystalline cellulose, and a tablet size may vary because tablets should be made with an excessive amount of mixture. Further, when maintaining the tablet size in this state, it is necessary to increase a tablet-punching pressure, which may affect disintegration due to an increase in the hardness. Furthermore, when tablet-punching, the surface is not smooth and a binding force is reduced, which in turn increases friability and may eventually cause a problem in tablet-punching ability and coating.

The pharmaceutical composition with improved stability, which includes the natural extract according to the present invention, may further include a pharmaceutically acceptable excipient, but does not include a solubilizer and a disintegrant.

The excipient may be a lubricant, and the lubricant may include any lubricant commonly used in the art, for example, silicon dioxide, magnesium stearate, and a mixture thereof, but it is preferable to apply silicon dioxide and magnesium stearate together. Further, the lubricant may be used in an amount of 2 to 4 wt.% based on the total weight of the pharmaceutical composition of the present invention.

On the other hand, the pharmaceutical composition with improved stability, which includes the natural extract according to the present invention, maybe formulated in the form selected from the group consisting of tablets, pills, powders, granules, capsules, suspensions, solutions, emulsions, syrups, and lyophilizing preparations. However, it is most preferable to formulate in the form of a tablet in consideration of the spirit of the present invention.

When the pharmaceutical composition is formulated in the form of a tablet, the tablet may include a coating layer, and the coating layer may include only an aqueous coating agent. That is, as described above, as the present invention secures stability against moisture by applying a specific amount of the crystalline cellulose and the lubricant, etc., the existing moisture-proof coating agent is not included (that is, the pharmaceutical composition may exhibit moisture-proof effects even without a moisture-proof coating agent, and such moisture-proof effects may be expressed by any one or more among a dextrin-based compound, crystalline cellulose and a lubricant).

The water-based coating agent may include, for example, hydroxypropylmethyl cellulose (HPMC), glycerin fatty acid ester, titanium dioxide, Lac pigment, and substances having properties and features similar or identical to the above materials. At this time, the hydroxypropylmethyl cellulose can be replaced with methyl cellulose, ethyl cellulose, hydroxypropyl cellulose, or a cellulosic compound having properties similar to the above materials. In addition, the water-based coating agent may further include any one or more of a solubilizer, a plasticizer, a binder, a lubricant, a wetting agent, a sweetener, a flavoring agent, an emulsifier, a suspending agent, and a diluent, if necessary.

The pharmaceutical composition of the present invention, which includes the above components, has various benefits in addition to the advantages described above. First, the pharmaceutical composition of the present invention may reduce the content of the drug since not using a solubilizer, a disintegrant, and a moisture-proof coating agent unlike the conventional ones. Further, in the case of a general tablet, the hardness is increased as the water content is decreased, such that the disintegration time also tends to be increased. However, in the case of a tablet formulated using the pharmaceutical composition of the present invention, the hardness is not increased as the moisture content is maintained, therefore, has an advantage that a time required for disintegration is constant within 30 minutes even without the use of a disintegrant (not affected by storage temperature and period).

Hereinafter, examples will be given to describe the present invention in detail.

### Preparative Example: Preparation of Chrysanthemum Extract

In order to obtain the Chrysanthemum extract, the Chrysanthemum extract was prepared using hot water and organic solvent extraction methods.

### 1. Hot water extraction method

In order to prepare a hot water extract of Chrysanthemum Sibiricum, after washing this natural plant with distilled water and finely pulverizing it with a grinder, the pulverized product was heated in a boiling water to obtain the Chrysanthemum extract.

At this time, after adding 1.0 L of water to each 100 g of Chrysanthemum raw material, it was extracted by heating in the boiling water at 100 to 120 °C for about 2 to 3 hours, and a cooler was installed to maintain a constant temperature and prevent loss by steam. The obtained Chrysanthemum extract was filtered to obtain a supernatant, followed by concentration using a concentrator. The Chrysanthemum extract powder was prepared by freezing the Chrysanthemum concentrate and lyophilizing the same at -80°C. As the drying method, in addition to the lyophilizing, vacuum drying or hot air drying may be used.

### 2. Organic solvent extraction method

In order to prepare an organic solvent extract (ultrasonic extract) of Chrysanthemum Sibiricum, this natural herb was immersed in 70% ethanol and extracted for about 2 to 3 weeks by an ultrasonic extractor to obtain an extract, then only the supernatant was separated and filtered through gauze to remove foreign substances. The alcohol extract obtained by the above process was concentrated using a concentrator and frozen, followed by lyophilizing at -80°C to prepare an extract powder of the Chrysanthemum extract. In this method, the ultrasonic extractor is used for the purpose of rapid extraction within a short time, but the ultrasonic extraction step may not necessarily be used in mass production. As in the hot water extraction method, vacuum drying or hot air drying may be used instead of lyophilization.

### Example 1. Human application test and results of ingestion of tablets containing Chrysanthemum extract

### 1. Human application test procedure

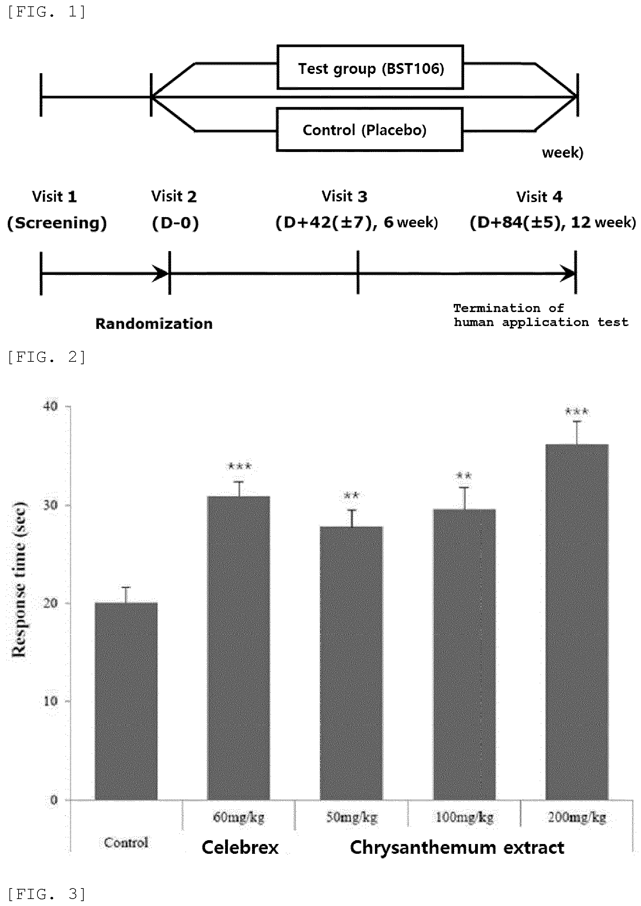

(1) Subjects of the human application test are randomly assigned to a test group or a control only for those who are suitable for human application test after determining whether the selection or exclusion criteria are met through a visit evaluation for people with mild degenerative knee arthritis symptoms. Then, the food for human application test (test food or control food) was supplied for ingestion for 12 weeks. The outline of the human application test is shown in FIG. 1.
(2) The constitutional composition of the test food is shown in Table 1 below, while that of the control food is shown in Table 2 below. In Table 1, the Chrysanthemum extract used herein was an ultrasonic extract prepared by the organic solvent extraction method described in the above preparative example.

**[TABLE 1]**

| **Name of raw material** | **Mixing ratio (%)** | **Content (mg)** |
|---|---|---|
| Chrysanthemum extract | 41.67 | 250.00 |
| Crystalline cellulose | 52.33 | 314.00 |
| Silicon dioxide | 1.80 | 10.80 |
| Magnesium stearate | 1.20 | 7.20 |
| HPMC | 2.07 | 12.41 |
| Glycerin fatty acid ester | 0.62 | 3.73 |
| Titanium dioxide | 0.21 | 1.24 |
| Lac pigment | 0.10 | 0.62 |
| **Sum** | **100** | **600** |

**[TABLE 2]**

| **Name of raw material** | **Mixing ratio (%)** | **Content (mg)** |
|---|---|---|
| Crystalline cellulose | 74.00 | 444.00 |
| Malto-dextrin | 22.00 | 132.00 |
| Magnesium stearate | 0.50 | 3.00 |
| Silicon dioxide | 0.50 | 3.00 |
| HPMC | 2.07 | 12.41 |
| Glycerin fatty acid ester | 0.62 | 3.73 |
| Titanium dioxide | 0.21 | 1.24 |
| Lac pigment | 0.10 | 0.62 |
| **Sum** | **100** | **600** |

(3) Before entering the human application test, past medical history and drug administration history were checked through past medical records and interviews.
(4) Each of the test group and the control ingested test food (250 mg/day as Chrysanthemum extract) and control food once a day for 12 weeks (84 days).

### 2. Experimental results

### (1) Comparison of TEARS levels

**[TABLE 3]**

| | n | Test group (N=53) | n | Control (N=57) |
|---|---|---|---|---|
| Standard (visit 1) | 53 | 9.16 | 57 | 9.18 |
| 12 week (visit 4) | 53 | 8.95 | 57 | 9.46 |

As shown in Table 3 above, it was demonstrated in the results of analyzing a change amount, i.e., variation of TBARS that the level thereof was decreased by 0.21 µM in the test group, whereas it was increased by 0.26 µM in the control after 12 weeks from ingestion.

In turn, it could be seen that the oxidative damage of synovial cells was improved in the test group in which the TBARS level was decreased, whereas the oxidative damage of the synovial cells was not improved in the control in which the TBARS level was increased.

### (2) Measurement of MMP-3 levels

Table 4 shows the results of analyzing the variation of MMP-3 by the PP set, and it was confirmed that after 12 weeks from ingestion, the level thereof was decreased by 0.76 ng/ml in the test group, whereas it was increased by 1.20 ng/ml in the control.

**[TABLE 4]**

| | Test group | | Control | |
|---|---|---|---|---|
| | N=53 | | N=57 | |
| | n | Mean | n | Mean |
| Standard (visit 1) | 53 | 18.75 | 57 | 18.03 |
| 12 week (visit 2) | 53 | 17.99 | 57 | 19.22 |
| Variation compared to standard | 53 | -0.76 | 57 | 1.20 |

Further, Table 5 shows the results of analyzing the variation of MMP-3 by the FA set, and it was confirmed that after 12 weeks from ingestion, the level thereof was decreased by 0.76 ng/ml in the test group, whereas it was increased by 1.20 ng/ml in the control.

**[TABLE 5]**

| | Test group | | Control | |
|---|---|---|---|---|
| | N=58 | | N=58 | |
| | n | Mean | n | Mean |
| Standard (visit 1) | 58 | 18.30 | 58 | 18.03 |
| 12 week (visit 2) | 57 | 17.96 | 58 | 19.22 |
| Variation compared to standard | 57 | -0.42 | 58 | 1.19 |

Since MMP-3 was decreased in the test group, whereas MMP-3 was increased in the control, it could be determined that the oxidative damage was improved in the test group ingesting the test food (including Chrysanthemum extract).

### Example 2. Assessment of analgesic efficacy of Chrysanthemum extract (Chrysanthemum 70% ethanol extract)

### 1. Experimental method

### (1) Standard

The experimental method used in this example is an analgesic efficacy experiment method using a hot plate test described in the "Guidelines for efficacy test of herbal preparations - General efficacy tests for painful diseases" announced by the Ministry of Food and Drug Safety, wherein the analgesic effect in the central nervous system of a mouse is measured with a response time to a heat stimulus as an index through a hot plate. At this time, the response time to the heat stimulus is measured as a time elapsed until the experimental animal licks the paw or jumps.

### (2) Drug administration

1) Celebrex (Celecoxib, Pfizer, 200 mg), Saline (Chungwae Pharmaceutical), Chrysanthemum extract (Green Cross HS) and male ICR mice (6 weeks old, weight 30-35 g, Orient Bio) were prepared. In this example, the Chrysanthemum extract was extracted by the organic solvent extraction method described in the above preparative example wherein it was extracted using 70% ethanol.
2) 60 mg/kg of Celebrex and 50, 100 and 200 mg/kg of Chrysanthemum extracts were prepared and orally administered to ICR mice, respectively. At this time, Celebrex is converted into administration concentration and suspended in saline while vortexing, and Chrysanthemum extract is suspended in 0.5% CMC and sonicated at 20 to 25 °C for about 10 minutes.

### 2. Assessment and results

### (1) Evaluation method

According to the efficacy experimental method using the hot plate test method, as the experimental animals, 5-week-old male ICR mice were purchased, acclimatized for 1 week, and then tested. The hot plate was maintained at 55°C, and drug administration was performed 30 minutes before this method. The central pain response due to heat was measured with an index, i.e., the time elapsed until the mouse licks the hind paw or jumps starting from contacting with the hot plate.

### (2) Classification of test groups (n=8)

**[TABLE 6]**

| | |
|---|---|
| 1 | Control (vehicle) |
| 2 | Celebrex 60 mg/kg, oral |
| 3 | Chrysanthemum extract 50 mg/kg, oral |
| 4 | Chrysanthemum extract 100 mg/kg, oral |
| 5 | Chrysanthemum extract 200 mg/kg, oral |

### (3) Experimental results

After administering the Chrysanthemum extract by concentration as shown in Table 6, and after 20 minutes, the mouse was placed on a hot plate maintained at 55 °C. Then, the pain response due to heat was measured with an index, i.e., the time elapsed until the mouse licks the hind paw or jumps starting from contacting with the hot plate. Measured results are shown in FIG. 2 and Table 7.

**[TABLE 7]**

| Respon se time (sec) | G1 Contr ol | G2 Celecox ib 60 mg/kg | G3 Chrysanthe mum extract 50 mg/kg | G4 Chrysanthe mum extract 100 mg/kg | G5 Chrysanthe mum extract 200 mg/kg |
|---|---|---|---|---|---|
| 1 | 24.44 | 32.71 | 30.41 | 25.33 | 28.06 |
| 2 | 18.20 | 25.61 | 35.08 | 29.46 | 35.12 |
| 3 | 15.81 | 26.94 | 22.16 | 22.12 | 30.78 |
| 4 | 18.52 | 27.82 | 26.14 | 38.12 | 34.30 |
| 5 | 24.51 | 35.55 | 31.69 | 28.08 | 41.17 |
| 6 | 24.71 | 36.22 | 20.86 | 32.95 | 38.92 |
| 7 | 13.42 | 28.54 | 28.74 | 22.56 | 32.85 |
| 8 | 20.92 | 33.71 | 27.21 | 37.80 | 48.24 |
| Mean | 20.07 | 30.89 | 27.79 | 29.55 | 36.13 |
| % of contro l | | 53.9 | 38.4 | 47.2 | 80.3 |

In the case of the control, the response time was 20.1 seconds. For the experimental groups administered with 50, 100, and 200 mg/kg of Chrysanthemum extract, the response time was increased significantly compared to the control, and the increase rates were confirmed to be 38.4%, 47.2%, and 80.3%, respectively. The Celebrex (including celecoxib) administration group as a positive control drug also showed an increase in the hot plate response time to 53.9%.

In turn, the response time was significantly increased in all dose groups to which the Chrysanthemum extract was administered, as compared to the control. In particular, the 200 mg/kg Chrysanthemum administration group had a greater response time compared to the Celebrex administration group. From these results, it could be seen that Chrysanthemum extract can relieve pain in patients by exhibiting a strong central nervous system writhing inhibitory activity.

### Example 3. Assessment of analgesic efficacy for Chrysanthemum extract (Chrysanthemum 70% ethanol extract) in acetic acid-induced writhing test model

### 1. Experimental method

### (1) Standard

The experimental method used in this example is the "Acetic acid writhing test" described in the "Guidelines for efficacy test of herbal preparations - General efficacy tests for painful diseases" announced by the Ministry of Food and Drug Safety, wherein acetic acid is administered intraperitoneally to cause damage of capillaries and pain in the abdominal cavity, and the analgesic effect in peripheral nerves is measured using the abdominal contractile response as an index.

### (2) Drug administration

1) Celebrex (Celecoxib, Pfizer, 200 mg), acetic acid (Daejunghwageum), Saline (Chungwae Pharmaceutical), Chrysanthemum extract (Green Cross HS) and male ICR mice (5 weeks old, Orient Bio) were prepared. In this example, the Chrysanthemum extract was extracted by the organic solvent extraction method described in the above preparative example wherein it was extracted using 70% ethanol.
2) 60 mg/kg of Celebrex and 50, 100 and 200 mg/kg of Chrysanthemum extracts were prepared and orally administered to ICR mice. At this time, Celebrex is converted into administration concentration and suspended in saline while vortexing, and Chrysanthemum extract is suspended in 0.5% CMC and sonicated at 20 to 25 °C for about 10 minutes.

### 2. Assessment and Results

### (1) Evaluation method

For the experimental method for analgesic effects to acetic acid-induced writhing, as the experimental animals, 4-week-old male ICR mice were purchased, acclimatized for 1 week, and then tested. The Chrysanthemum extract and control drug, respectively, were orally administered to mice by dose, followed by intraperitoneal injection of acetic acid (0.7% acetic acid-physiological saline (0.1 ml/10 g BW)) 30 minutes after administration. Subsequently, from 5 minutes after injection, the number of occurrences of writhing was measured for 10 minutes (FIG. 3) .

### (2) Classification of test groups (n=6)

**[TABLE 8]**

| | |
|---|---|
| 1 | Control |
| 2 | Celebrex 60 mg/kg, oral |
| 3 | Chrysanthemum 50 mg/kg, oral |
| 4 | Chrysanthemum 100 mg/kg, oral |
| 5 | Chrysanthemum 200 mg/kg, oral |

### (3) Experimental results

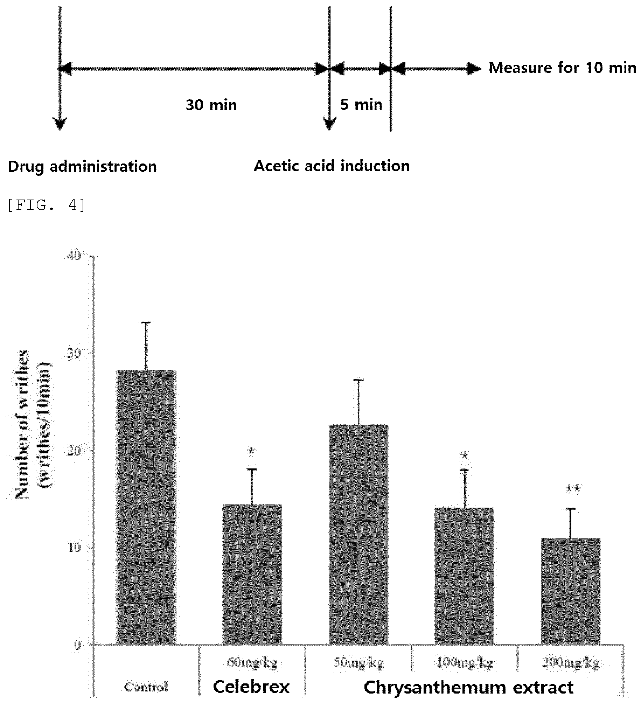

The Chrysanthemum extract was administered by concentration as shown in Table 8 and, after 30 minutes, 0.7% acetic acid-saline was administered intraperitoneally to induce pain. Subsequently, from 5 minutes after the intraperitoneal administration, the number of occurrences of writhing syndrome was counted for 10 minutes and results thereof are shown in FIG. 4 and Table 9.

**[TABLE 9]**

| Writhing syndrome /10 min | G1 Contr ol | G2 Celecox ib 60 mg/kg | G3 Chrysanthe mum extract 50 mg/kg | G4 Chrysanthe mum extract 100 mg/kg | G5 Chrysanthe mum extract 200 mg/kg |
|---|---|---|---|---|---|
| 1 | 23.00 | 25.00 | 19.00 | 9.00 | 3.00 |
| 2 | 24.00 | 18.00 | 17.00 | 3.00 | 6.00 |
| 3 | 27.00 | 9.00 | 27.00 | 20.00 | 12.00 |
| 4 | 28.00 | 6.00 | 22.00 | 27.00 | 23.00 |
| 5 | 40.00 | 15.00 | 29.00 | 12.00 | 11.00 |
| 6 | 28.00 | 14.00 | 22.00 | 14.00 | 11.00 |
| Mean | 28.33 | 14.50 | 22.67 | 14.17 | 11.00 |
| % of control | | 48.9 | 20.2 | 50.1 | 61.3 |

In the case of the control, the number of occurrences of writhing syndrome was 28.33/10 min, while the group administered with 100 and 200 mg/kg Chrysanthemum extract, excluding the group administered with a low dose of 50 mg/kg of Chrysanthemum extract, had the number of occurrences of writhing syndrome reduced by 50.1% and 61.3%, respectively, compared to that of the control. The group administered with Celebrex (including celecoxib) as a positive control also had the number of acetic acid-induced writhing syndromes significantly reduced, and the inhibition rate was confirmed to be 48.9%.

From the above results, it was confirmed that the number of occurrences of writhing syndrome was significantly reduced in all dose groups administered with the Chrysanthemum extract compared to the control, and the number of occurrences of writhing syndrome was further significantly reduced in the 100 mg/kg and 200 mg/kg Chrysanthemum extract administration groups compared to the Celebrex administration group. Therefore, it could be determined that the Chrysanthemum extract can relieve the pain of the patient by exhibiting a strong peripheral nervous system pain inhibitory activity.

## Claims

1. A health functional food for pain relief or antioxidation, comprising Chrysanthemum Sibiricum extract ("Chrysanthemum extract").

2. The health functional food according to claim 1, wherein the antioxidation is to improve oxidative damage of synovial cells.

3. The health functional food according to claim 2, wherein the oxidative damage is damage in a patient with degenerative arthritis.

4. The health functional food according to claim 1, wherein the food is used to prevent or improve a disease selected from the group consisting of: Alzheimer's disease, Parkinson's disease, Huntington's disease, amyotrophic lateral sclerosis, multiple sclerosis, obesity, hyperglycemia, dyslipidemia, hypertension, diabetes mellitus, heart failure, cardiovascular ischemia, cerebral ischemic injury, stroke, myocardial infarction, systemic lupus erythematosus, COPD, asthma, acute kidney injury, chronic kidney disease, diabetic nephropathy, end-stage renal disease, viral hepatitis, liver cirrhosis, inflammatory bowel disease, viral infectious disease, solid cancer, hematologic cancer, sepsis, recurrent polychondritis, Kienbock's disease in adults, osteochondrosis of the carpal meniscus, complex regional pain syndrome, Paget's disease, progressive ossified fibrodysplasia, relapsing steatosis, multifocal fibrosclerosis, diffuse Fasciitis, polymyalgia rheumatica, Behcet's disease, mixed connective tissue disease, Sjegren's syndrome, dry syndrome, systemic sclerosis, Crest's syndrome, polymyositis, dermatomyositis, systemic lupus erythematosus, aortic arch syndrome, Wegener's granulomatosis, lethal median granuloma, thrombotic thrombocytopenic purpura, thrombotic microangiopathy, Goodpasture's syndrome, polyarteritis, polyarthritis, juvenile arthritis, juvenile rheumatoid spondylitis, rheumatoid arthritis and adult-onset Still's disease.

5. The health functional food according to claim 1, wherein the Chrysanthemum extract is a hot water extract or an organic solvent extract.

6. The health functional food according to claim 1, wherein the pain is selected from the group consisting of: somatic pain, visceral pain, inflammatory pain, dysfunctional pain, idiopathic pain, neuropathic pain, superficial pain, deep pain, itching, migraine and cancer pain.

7. A pharmaceutical composition for treatment or prevention of a disease, comprising Chrysanthemum Sibiricum extract ("Chrysanthemum extract"), wherein the disease is selected from the group consisting of: Alzheimer's disease, Parkinson's disease, Huntington's disease, amyotrophic lateral sclerosis, multiple sclerosis, obesity, hyperglycemia, dyslipidemia, hypertension, diabetes mellitus, heart failure, cardiovascular ischemia, cerebral ischemic injury, stroke, myocardial infarction, systemic lupus erythematosus, COPD, asthma, acute kidney injury, chronic kidney disease, diabetic nephropathy, end-stage renal disease, viral hepatitis, liver cirrhosis, inflammatory bowel disease, viral infectious disease, solid cancer, hematologic cancer, sepsis, recurrent polychondritis, Kienbock's disease in adults, osteochondrosis of the carpal meniscus, complex regional pain syndrome, Paget's disease, progressive ossified fibrodysplasia, relapsing steatosis, multifocal fibrosclerosis, diffuse Fasciitis, polymyalgia rheumatica, Behcet's disease, mixed connective tissue disease, Sjegren's syndrome, dry syndrome, systemic sclerosis, Crest's syndrome, polymyositis, dermatomyositis, systemic lupus erythematosus, aortic arch syndrome, Wegener's granulomatosis, lethal median granuloma, thrombotic thrombocytopenic purpura, thrombotic microangiopathy, Goodpasture's syndrome, polyarteritis, polyarthritis, juvenile arthritis, juvenile rheumatoid spondylitis, rheumatoid arthritis and adult-onset Still's disease.

8. The pharmaceutical composition according to claim 7, wherein the pain is selected from the group consisting of: somatic pain, visceral pain, inflammatory pain, dysfunctional pain, idiopathic pain, neuropathic pain, superficial pain, deep pain, itching, migraine and cancer pain.

9. The pharmaceutical composition according to claim 7, wherein the prevention or treatment is based on antioxidation by reduction of Thiobarbituric acid reactive substance (TBARS).

10. The pharmaceutical composition according to claim 9, wherein the antioxidation is to improve oxidative damage of synovial cells.

11. The pharmaceutical composition according to claim 7, wherein the Chrysanthemum extract is a hot water extract or an organic solvent extract.
